# NEW EUROPEAN PATENT SPECIFICATION

(11) **EP 0 906 414 B2**
(45) Date of publication and mention of the opposition decision: **12.08.2015**
(45) Mention of the grant of the patent: 10.09.2003
(21) Application number: 97916373.0
(22) Date of filing: 21.03.1997
(51) Int. Cl.: C12N 1/12, C12N 1/14, C12P 7/64, C12N 1/00

(54) **PROCESS FOR THE PREPARATION OF A GRANULAR MICROBIAL BIOMASS AND ISOLATION OF VALUABLE COMPOUNDS THEREFROM**
VERFAHREN ZUR HERSTELLUNG VON GRANULÄRER MIKROBIELLER BIOMASSE UND GEWINNUNG WERTVOLLER KOMPONENTEN AUS MIKROBIELLER BIOMASSE
PROCEDE POUR LA PREPARATION D'UNE BIOMASSE MICROBIENNE GRANULAIRE ET ISOLATION DE COMPOSES INTERESSANTS A PARTIR DE CETTE DERNIERE

(30) Priority: 28.03.1996 EP 96200837
(43) Date of publication of application: 07.04.1999
(60) Divisional application: 03019664.6
(73) Proprietor: DSM IP Assets B.V., 6411 TE Heerlen (NL)
(72) Inventor: BIJL, Hendrik, Louis, NL-3131 ZD Vlaardingen (NL); SCHAAP, Albert, NL-2993 BG Barendrecht (NL); VISSER, Johannes, Martinus, Jacobus, NL-2614 XC Delft (NL)
(74) Representative: Elkenbracht, Johan Christiaan
(86) International application number: PCT/EP1997/001446
(87) International publication number: WO 1997/036996

(56) References cited:
- EP-A- 0 322 227
- EP-A- 0 688 871
- EP-A2- 0 046 017
- EP-A2- 0 202 409
- WO-A-91/11918
- WO-A-92/12711
- WO-A-93/20183
- WO-A-94/29292
- WO-A1-86/04354
- WO-A1-88/08025
- DD-A- 150 627
- DD-A- 150 627
- DE-A- 1 923 529
- FR-A- 2 210 662
- FR-A1- 2 599 597
- GB-A- 882 634
- GB-A- 1 411 450
- GB-A- 1 466 853
- KR-A- 830 002 437
- US-A- 3 297 731
- US-A- 3 958 027
- US-A- 4 056 638
- US-A- 4 056 638
- US-A- 4 646 631
- US-A- 5 340 594
- US-A- 5 374 657
- US-A- 5 539 133
- JAREONKITMONGKOL S ET AL: "PRODUCTION OF DIHOMO-GAMMA-LINOLENIC ACID BY A DELTA-5 DESATURASE-DEFECTIVE MUTANT OF MORTIERELLA-ALPINA 1S-4" APPLIED AND ENVIRONMENTAL MICROBIOLOGY, 58 (7). 1992. 2196-2200., XP000676362
- JAREONKITMONGKOL S ET AL: "PRODUCTION OF AN EICOSAPENTAENOIC ACID-CONTAINING OIL BY A DELTA-12 DESATURASE-DEFECTIVE MUTANT OF MORTIERELLA-ALPINA 1S-4" JOURNAL OF THE AMERICAN OIL CHEMISTS' SOCIETY, 70 (2). 1993. 119-123., XP000676339
- DATABASE WPI Week 9210 Derwent Publications Ltd., London, GB; AN 92-077323 XP002035614 & JP 04 023 979 A (KOBE STEEL LTD) , 28 January 1992
- KYLE D J; RATLEDGE C ED.: "Industrial applications of single cell oils." 1992 , AMERICAN OIL CHEMISTS SOCIETY , CHAMPAIGN XP002044902 see page 61; figures 5-14
- RITTNER H. ET AL: 'Conditioning of Oil-Bearing Materials for Solvent Extraction by Extrucion' JAOCS vol. 61, no. 7, July 1984, pages 1200 - 1203
- 'Fats and Oils' YUSHI vol. 39, no. 4, 1986,
- SHINMEN, Y.: 'Production of polyunsaturated fatty acids by microorganisms', 1991 page 85
- Kokai Jap. unexamined patent application No: 2-171127
- HUI, Y.H.: 'Bailey's industrial oil and fat products', vol. 5, 1996 pages 61,84-89,106-107 - 114-119
- KYLE & RATLEDGE: '"Industrial applications of single cell oils"', 1992 page PREFACE PAGE

## Description

### Field of the invention

The present invention relates to a process for treating microbial biomass (generally a micro-organism-containing product resulting from fermentation) which may allow the isolation of useful or valuable compound(s) from the resulting biomass to be improved. The process can involve granulating the biomass (e.g. by extrusion) before recovery of the compound(s).

### Background of the invention

For some time now microorganisms have been known as valuable sources for a varied range of products. Several of these microbial products are located inside, or are associated with, the microbial cell. To recover such products in a relatively pure form, it is necessary to separate the product from the microbial biomass. For instance, in order to isolate lipophilic compounds from microbial biomass, usually an extraction (leaching) step with an organic solvent or a supercritical fluid (e.g. liquid CO₂) is performed.

The biomass results from fermentation and can be used for product extraction. It is usually a wet cell cake but various pretreatments cause significant disruption of the cells. Cell disruption can occur by physical treatment (e.g. drying, such as spray drying or lyophilization, and/or mechanical disintegration (such as by homogenisation or milling), by chemical (acid or alkali) or by enzymatical treatment. Drying of the biomass is desirable to reduce the amount of solvents and, in the case of extraction of lipophilic compounds, to prevent troublesome emulsions.

Usually spray drying is used to obtain biomass which is then ready for extraction. The drying by spray-driers requires the following conditions: in order to feed the dryer, the biomass should be a (concentrated) liquid or slurry with small particles (to enable the spraying by a disc or a nozzle). This means that for a microbial biomass the maximum dry matter content of the slurry is limited to approximately 20-30%, resulting in a subsequent drying step, to reach a dry matter content of 90-95%, that will be relatively expensive.

The spray drying process is relatively short in terms of time. However, the product temperature in most cases is (relatively) high. This means that there is a high oxidation and subsequent degradation risk for oxidation-sensitive compounds. In case of a biomass with high amounts of intracellular lipid material, the drying is sometimes troublesome due to "sweating" of the lipid from the biomass at higher temperatures. In this case fouling of the dryer will occur.

Another drawback of spray drying is that this significantly limits the type of extraction process which can be used subsequently to isolate a desired compound. It is usually impossible to apply, for example, percolation extraction techniques on fine-powdered products such as spray dried biomass. Mechanical disruption of the microbial biomass, such as by milling, also has the same drawback of producing a large amount of fines (or dust).

UK patent no. GB 1 466 853 relates to a process to isolate oil from spray-dried yeast powder. The yeast powder is wetted and heated by steam at 80°C in a cooker. The wet powder was then fed into a palletising machine, converted to pellets, flaked and dried again to reduce the moisture content to 5-10% by weight. The resulting flakes then were filtered by a screen and the were recycled to the pelletiser. This material was suitable for percolation extraction. However, the method has the disadvantage that the powder, once it has been spray-dried, needs to be rehydrated, treated at high temperatures and then redried. For thermo- and/or oxidation-sensitive compounds, such adverse treatment steps results in degradation and hence poor yields.

International patent application WO-A-93/25644 (equivalent to US 5,539,133) refers to a method for the isolation of lipids with a high content of polyunsaturated fatty acids (PUFAs) from macro- and/or micro-algae (a type of seaweed), where times the macroalgae can be residues from alginate or carrageenan extraction. The material derived from algae have particle sizes of less than 50 mm and a dry matter content of more than 50%. With algal material having a particle size larger and/or a dry matter content less than the indicated values, the material needs to be pretreated by milling and/or drying. However, as indicated above, milling is disadvantageous for an efficient extraction and refining process.

DD-A-150627 refers to preparing a yeast (or bacteria) containing biomass which is dried, and then made into large granules (8-12 mm, apparently spherical). These have a water content of 20%, and are then milled. Extraction is performed using petroleum ether to reclaim unwanted diesel (or hydrocarbons) that were used as a carbon source for the microorganisms. The remaining residue is used as an animal feed.

DE-A-1923529 refers to a biomass which is subjected to evaporation before being dried on a fluid bed to produce granules. These are then milled or crumbled. The residual oil (as a contaminant) is then extracted. In common with the previous document, the substance being extracted is an impurity, and is not produced by the microorganisms contained in the biomass.

EP-A-0322227 refers to a process for production of arachidonic acid (ARA) and gamma-linoleic acid (GLA) by feeding a microorganism with sesame oil.

US patent no. 5,340,594 refers to the production of highly unsaturated fatty acids from fungi. The biomass can be mixed with ground corn, and the resulting product used as an animal feed.

WO-A-92/12711 refers to blends of microbial oils, particularly as supplements for infant formula, where the biomass is filtered and squeezed on a filter to produce a filter cake, which is then freeze dried.

The present invention seeks to provide a process of extraction of desired compounds from a biomass that may that solve some or all of problems present in the prior art.

### Description of the invention

According to a first aspect of the present invention, there is provided a process for the isolation of one or more compound (s) from a microbial biomass comprising a microorganism that has produced one of the compound(s), the process comprising:
a) providing, or obtaining, a biomass with a dry matter content of from 25% to 80%;
b) extruding the biomass into granular particles having an average dry matter content of from 25% to 80%;
c) drying the granular particles to give porous dried granules having an average dry matter content of at least 80%; and
d) purifying, extracting or isolating the or each compound from the dried granules resulting from (c),
   wherein
A) the biomass comprises, or originates from, a filamentous fungus or an algae or the microorganism is a bacteria; or
B) in d), the or each compound is extracted from the dried granules resulting from (c) using a solvent which is a C₁₋₁₀ alkyl ester, toluene, a CH₁₋₆ alcohol or a CH₃₋₈ alkane.

It has been surprisingly found that by using dried granules of the biomass a higher yield than expected of the compound to be isolated can be achieved. This is thought to be due to the structure of the granules which can maximise access of the solvent to be used for the extraction. Of course, if the particles are too large, then the surface area may be lower, resulting in a correspondingly lower yield. However, the particles should not be too small otherwise they may clog the filter that is used during extraction. For this reason, the process of the invention does not include a milling, flaking or comminuting step or stages.

The water content at various stages can also influence yields. Too high a dry matter content, and the biomass will crumble and may form fines or dust, which is disadvantageous if a filtration extraction method is employed. However, too high a water content and one obtains a slurry that is too wet to be made into granules.

Processes for granulating matter are known in the art. However, they are often combined with milling or flaking at some stage, which gives the disadvantages as discussed above. In the present invention, it is the dried granules that are used for extraction of the compound, and not a milled or flaked form. In addition, by granulation, damage to the cells in the biomass may be minimized, which again can help increase yields of the compound. In US 5,340,594 extrusion of a biomass is disclosed, but here the extruded form is used as an animal feed: there was no appreciation that the granular form would give high yields on extraction of a particular compound from that granular form.

By processing the biomass into granular particles, one can assist the drying process. Drying can be considerably easier and more efficient after the biomass has been processed into a granular form.

In addition, the dried granules have been found to be particularly stable, especially at ambient or room temperatures. The biomass can be stored for a considerable length of time in this form, without degradation. Although not wishing to be bound by theory, it is suspected that this occurs because the compound is located inside the granules and therefore at least partially protected from the environment, which can, for certain compounds, cause degradation by oxidation.

The preferred method of granulating the biomass is by extrusion. This can minimise destruction of the cells. The stability of the biomass has been found to be better with minimum disruption of the cells, in other words the process of the invention can be adapted to optimize the number of cells that remain intact. This is in contrast to many prior art extractions, where the cells are disrupted in order to isolate the compound.

A second aspect of the present invention relates to a process for the isolation of one or more compound(s) from granules of biomass, the process comprising:
a) providing porous dried granules having a dry matter content of at least 80%, the granules having been derived from an extruded microbial biomass comprising microorganisms that have produced such a compound; and
b) extracting or isolating the or each compound from the dried granules by solvent extraction,
   wherein
   A) the biomass comprises, or originates from, a filamentous fungus or an algae or the microorganisms are bacteria; or
   B) the extraction in b) is conducted using a solvent which is a C₁₋₁₀ alkyl ester, toluene, a CH₁₋₆ alcohol or a CH₃₋₈ alkane.

The preferred extraction method is to employ a solvent, in which suitably the compound is soluble. The preferred extraction method is to use percolation: here the solvent can be passed over a bed of the granules. For this technique it will be appreciated that the particles should not be too small (for example they should not be milled or comminuted) otherwise one will obtain too much "dust" (or fines) which will clog the filter. Large particles are also to be avoided, but in between these two extremes one can obtain an optimal surface area, so that preferably the granules are larger than the pores of the filter. The particles are preferably highly porous to allow easy access of the solvent to the compound to be extracted.

In the present invention the pretreatment of microbial biomass cake to form granular particles can significantly improve the subsequent drying process. The resulting dried granulated biomass can be particularly suitable for either immersion or percolation extraction. The particle size can be specifically adjusted for optimal drying and extraction conditions. By using biomass pretreated according to the invention, the desired compound is advantageously extracted without the need to disrupt the cells prior to extraction.

The process of the invention can be used to prepare granular particles or dried granules from almost any type of microorganism. The microorganism can be in a filamentous form, like fungi or certain bacteria, or as single cells, like yeasts, algae and bacteria. Thus, the biomass may comprise microorganisms that are yeasts, fungae, bacteria or algae. Preferred fungae are of the order *Mucorales.* For example, the fungus may be of the genus *Mortierella, Phycomyces, Blakeslea,* or *Aspergillus.* Preferred fungae are of the species *Mortierella alpina, Blakeslea trispora* and *Aspergillus terreus.*

As far as yeasts are concerned, these are preferably of the genus *Pichia,* such as of the species *Pichia ciferrii*.

Bacteria can be of the genus *Propionibacterium*.

If the biomass comprises an algae, this is preferably a dinoflagellate and/or belongs to the genus *Crypthecodinium*. Preferred algae are of the species *Crypthecodinium cohnii.*

The compound to be isolated from the microbial biomass prepared according to the invention may be located intracellularly, associated with the cell membrane or cell wall, or produced extracellularly (it may then be insoluble in water).

The compound to be isolated can be either hydrophilic or hydrophobic (e.g. lipophilic). Examples of such compounds are intracellular proteins or enzymes, lipids, secondary metabolites like vitamins (e.g. vitamin B₁₂), macrolide or polyene antibiotics, flavour providing substances or carotenoids. Preferably, the compound to be isolated from microbial biomass is a lipophilic compound.

The compound extracted from the biomass treated according to the invention can be of high quality since it has been subjected to little, if any, deterioration due to the mild conditions used in the treatment process. Therefore, the invention is particularly suitable for the preparation of microbial biomass from which heat- and/or oxidation-sensitive compound(s) need to be isolated.

The invention is suitable for preparing microbial biomass for the isolation of compounds having a high degree of unsaturation, such as lipids containing polyunsaturated fatty acids (PUFA). Preferably the PUFA is a C18, C20 or C22 ω-3 or ω-6 polyunsaturated fatty acid. For instance the compound can be docosahexaenoic acid (DHA) (from algae or fungi, such as the dinoflagellate *Crypthecodinium* or the fungus *Thraustochytrium*), γ-linolenic acid (GLA), dihomo-γ-linolenic or arachidonic acid (ARA) (from fungi, such as *Mortierella, Pythium* or *Entomophthora*), or eicosapentaenoic acid (EPA) (from algae, such as *Porphyridium* or *Nitzschia*). Any of these PUFAs may be isolated either on their own or, more usually, in the form of a lipid.

Additional examples of compounds which can be isolated according to the invention include β-carotene, such as from fungal genera e.g. from the order *Mucorales*, e.g. *Phycomyces* or *Blakeslea,* astaxanthin from the yeast *Phaffia rhodozyma*, tetraacetylphytosphingosine (TAPS) from the yeast *Pichia ciferrii*, and/or vitamin B12 from propionic bacteria.

Other compounds that can be extracted include lipophilic/non polar ones such as lovastatin, cyclosporin and laidlomycin. Of these, the first two are either produced extracellularly or attached to the cell wall. Suitable solvents, therefore, include heptane, hexane, acetone, methanol and toluene, and ethanol. However, for the later two compounds, one can use isopropyl alcohol or butyl acetate for cyclosporin, and ethanol or methanol for laidlomycin. Generally speaking, hexane is suitable for apolar antibiotics, such as those produced by the organisms of the genus *Streptomyces.*

Other compounds include polyketides, or metabolites derived from polyketides, which includes many antibiotics. Preferred polyketides are those that do not contain nitrogen, and may be aromatic, preferably containing at least one 6 membered ring. Preferred polyketides are statins, which includes lovastatin, simvastatin, pravastatin and compactin. Other preferred compounds are HMG-CoA reductase inhibitors. These can reduce cholesterol levels in the blood.

Another class of compounds that can be extracted include steroids, such as ergosterol. These are produced by yeasts and moulds.

The process of the invention is particularly suitable for the isolation of lipophilic compounds, such as polyunsaturated fatty acid(PUFA)-containing lipids and/or carotenoids.

The compounds isolated according to the process(es) compositions, of the invention are suitable for use in human or animal foods (e.g. infant formula) or other edible compositions and in cosmetics, healthcare compositions or supplements, or pharmaceutical compositions.

In the process of the invention, the microorganism of choice can first be fermented to obtain a sufficient amount of biomass for subsequent extraction of the compound. The fermentation conditions will depend on the organism used, and may be optimized for a high content of the compound in the resulting biomass.

Suitable fermentation conditions are presented in Comparative Example 26.

After the fermentation process has finished, the fermentation broth, depending on the type of compound to be isolated, may be pasteurized to kill the production organism and to inactivate any undesirable enzymes. If desired, flocculation agents and/or other processing aids may be added to the broth to improve its filterability.

Suitable flocculating agents include CaCl₂, Al₂(SO₄)₃ and polar cationic polyamides. These may be present at from 0.1 to 2% by weight.

Preferably the biomass (or broth) is pasteurised. After fermentation pasteurisation may be necessary to obtain a slurry that can be processed in a hygienic way. The pasteurisation of biomass in the fermenter can have several advantages. Firstly, the production organism is not exposed to the environment. Also, unwanted enzymatic activities, influencing the quality of the target compound can be inactivated.

Depending on the species of the production organism the pasteurisation is performed at temperatures of from 60 to 100 °C. The pasteurisation can be performed by heating (directly) with steam into the fermenter or by (indirect) heating using a medium via heat exchangers, either through the wall or with cooling coils or an external heat exchanger such as known plate heat exchangers or other suitable heat exchangers.

The following preferred pasteurisation conditions can be employed, especially for organisms of the genus *Mortierella*.

The fermentation broth (or biomass) is pasteurized to kill the microorganism and to inactivate enzyme activity. This can be about 144 hours after inoculation of the main fermenter. The biomass (or broth) is suitably pasteurized at from 50 to 95 °C, preferably from 60 to 75 °C, and optimally between 63 to 68 °C. This can be for from 30 to 90 minutes, preferably from 50 to 75 minutes, optimally, from 55 to 65 minutes. This can be by any suitable heating means, but is preferably by direct steam injection, such as into the main fermentation vessel.

After pasteurisation the broth is allowed to cool, or is cooled down. This can take about 4 hours, suitably to about 25 °C.

If two or more organisms are involved, from different biomass or fermentation broths, then each biomass (or broth) can be individually pasteurised or, after mixing, they can then be pasteurised. However, the former is preferred as different pasteurisation conditions can then be employed for the different organisms.

Usually, pasteurisation will take place in the fermenter vessel in which fermentation has occurred. However, for some organisms (such as bacteria) it is often preferred to remove the microorganisms from the vessel first, and then pasteurise (for example, before spray drying in an agglomeration granulation process).

Generally speaking, pasteurisation is advantageous because not only may it kill the microorganism, but more importantly it can inactivate one or more enzymes that can adversely affect the compound. For example, pasteurisation may inactivate various lipases, and these may cleave fatty acids off a triglyceride backbone. This is disadvantageous for PUFAs where a high triglyceride content is preferred.

As will have been appreciated, pasteurisation will usually kill most, if not all, of the microorganisms. Therefore, in the dried granules, at least 95%, such as at least 98%, if not 99%, of the microorganisms, have been killed (i.e. are not alive).

For some organisms (e.g. *Pichia*) preferably no pasteurisation is conducted.

To prevent recontamination of pasteurised biomass during subsequent processing steps conditions can be designed to reduce the risk of growth. One possibility is to acidify the broth with a suitable acid. To prevent the out-growth of many microbial species a pH range of from 3 to 4 in combination of a low process temperature is sufficient.

Also other biostatic agents like alcohols, sorbates, etc. may be used for this purpose.

For thermally stable products processing at higher temperatures (60 - 100 °C) may be applied.

Preferred acidifying conditions (e.g. for organisms of the genus *Mortierella*) are as follows.

The pH of the pasteurised broth is adjusted to from 2 to 5 to improve microbiological stability, preferably to a pH in the range of 3 to 4, and optimally a pH of about 3.5.

Acidification of the broth (before or after pasteurisation) can have additional advantages. If the compound is a polyketide, for example a statin, then acidification can result in precipitation of the compound. For many compounds, especially water soluble ones, precipitation before further processing steps is desirable, lest the compound be lost when the broth is filtered to remove water. Therefore, before or after pasteurisation, a compound may be precipitated (such as by acidification, although any other means known to a person skilled in the art can be employed).

The pH can be adjusted by any suitable means e.g. 85 % phosphoric acid, preferably diluted 55 % phosphoric acid and optimally with diluted 33 % phosphoric acid.

At this stage one has a broth, which may have been pasteurised. The next stage is to obtain a biomass, by separating the microorganisms from the surrounding medium.

A solid-liquid separation technique can be performed to separate the biomass from the fermentation broth. This (harvested) biomass usually has a dry matter content varying from 20 to 35%, depending on the type of microorganism. However, for extrusion (and subsequent drying) the biomass typically should have a dry matter content which ranges from 25% to 80%.

If the water content of the biomass is too high (e.g. for extrusion and/or subsequent drying), it can be dewatered and/or have its dry matter content increased. This can be achieved by a number of methods. Firstly, the biomass can be subjected to (additional) dewatering. Any dewatering method known to the skilled person can be used; the desired dry matter content can be from 25 or 30 to 80%.

Preferably, a mechanical dewatering method is used. The maximum dry matter content which can be reached by mechanical dewatering will, however, vary depending on the type of microorganism. For certain microorganisms, e.g. yeast, the dry matter content of the biomass after mechanical dewatering may not exceed a level of 35 to 40%, while the same process executed on biomass of certain lipid-rich microorganisms may result in a higher dry matter content of from 45 to 60%.

A preferred method is to use a membrane filter press (plate and frame filter press with squeezing membranes) which can combine a solid-liquid separation with mechanical dewatering and is especially suitable to obtain the desired dry matter content.

Alternatively or in addition, the desired dry matter content of the microbial biomass can be increased by the addition of consistency-increasing (or dry) agents. These consistency-increasing agents are suitably dry and, preferably, do not negatively interfere with the extraction process and/or the properties of the compound. For example, consistency-increasing agents can comprise starch and/or plant fibres such as oats or wheat bran or cellulose. Even another biomass (of a lower water content) can be used. Such substances may be added anyway, if it improves the extrudability.

Sometimes, e.g. after solid-liquid separation and/or mechanical dewatering, the biomass can form of large cakes. This may not be suitable for granulation (e.g. extrusion). To reduce the biomass to a size which may enable granulation (e.g. efficient feeding of the extruder), the biomass is suitably crumbled, kneaded and/or mixed. This crumbling and/or kneading can be achieved by (short) treatment in a high shear mixer. Optionally, the or each consistency-increasing agent may be added during this part of the process.

The then (optionally crumbled or kneaded) biomass can be subsequently subjected to the granulation process to result in the formation of granular particles. The granulation can be effected in a number of different ways.

Another method of reducing water content (or increasing dry matter content) is to use a salt (e.g. brine) wash, either of the biomass or (preferably) after separation of the biomass from the broth, such as using wash filtration.

The desired particle structure and size is obtained by an extrusion process. The particle characteristics, such as structure and size, can be important in order to optimise the drying and/or extraction process. During the drying step, if the particles are too small they may give problems as they can generate dust and fines, whereas too large particles do not fluidize and may give a poor drying performance. During extraction, a too small granule size may not allow the use of a percolation process, since the pressure drop over the biomass bed will be too high. Too much fines may give problems in subsequent purification steps. A too large size may impede efficient penetration of solvent during extraction. Furthermore, the particle structure should be sufficiently compact in order to prevent disintegration during drying and extraction, but the particles (dried granules) preferably have a porosity that allows (efficient) penetration of solvent during extraction.

The extrusion conditions can be adjusted by a skilled person in order to obtain granular (biomass) particles having the desired structure and size.

The extrusion conditions can be adjusted to minimize cell disruption. Minimal cell disruption can ensure optimal protection of labile, oxidation-sensitive' compounds against oxidation-induced degradation. Extrusion is therefore preferably conducted at lower temperatures, without any means of heating. Preferably this is in the range of from 20 to 30°C, such as about room temperature. During extrusion the granular particles may form naturally, the "extrudate" falling away under its own weight from the die plate by the influence of gravity, thereby forming particles. If, however, the biomass is of a nature whereby after being extruded by the die plate in forms long strands like spaghetti, then the spaghetti can be cut to give particles of a desired size.

The temperature of the biomass has been found to influence the nature of the granular particles produced on extrusion. Preferably the biomass has a temperature of from 6 to 15°C before extrusion. However, while in the extruder the temperature of the biomass can rise to be from 10 to 60°C, although preferably this is from 15 to 30°C. The temperature rise will depend upon the pressure exerted on the biomass, and its dry matter content.

During extrusion the biomass is usually forced through a barrel towards a die plate, often by a screw. This barrel is preferably not heated. In fact, it is advantageous that it is cooled. Suitably, the temperature of the coolant (e.g. an aqueous solution such as water) is from 1 to 4°C, such as about 2°C.

Generally speaking, extrusion does not change the water content. This is why in stage (b), the dry matter content is the same as in stage (a). However, as will be appreciated, other granulation techniques (such as those described later) do change the water content, and can decrease it (in other words, increase the dry matter content). For a biomass that contains a fungus, for example, of the order *Mucorales* (in particular one producing a PUFA) the dry matter content of the biomass in (a), which will usually be the same as in the granular particles produced on granulation (in this case extrusion) is suitably between 35 and 60%, preferably from 50 to 60%. After drying, the dry granules preferably have a dry matter content of at least 90%, such as at least 95%.

The preferred granulation technique is to use an extruder. A good overview of extruders is by W. Pietsch ("Size Enlargement by Agglomeration": Wiley & Sons, 1991, page 385). The machine maybe a batch or continuous extruder. For continuous extruders there may be mentioned simple single screw extruders (both axial and radial transporting). Also there are twin screw extruders either co-or counter rotating. The to be extruded biomass is transported, partly compacted and pressed through a perforated (die) plate. Another group of extruders include pelletising machines. Here a cylindric pressing tool rolls over a layer of material deposited on a perforated plate.

If the granules are obtained by extrusion, then the biomass needs to be in an extrudable form. The water content can be adjusted, if necessary, depending on the condition of the biomass, the microorganisms employed, and the extrusion conditions. Water can either be removed, or the dry matter content increased by means of addition of solids, for example starch. The biomass can in this way be adjusted to the correct consistency, which is usually that of a paste.

Although the granules can be used for extraction of the compound, they do in addition represent a stable form of the biomass that can be stored. The granules can have other uses: for example, they may be used in the preparation of an infant formula, where the biomass contains one or more polyunsaturated fatty acids (PUFAs).

The present invention also envisages other granulation methods which enable the formation of (granular) particles. For instance, a multistage drying process can comprise a combination of spray-drying and a fluidized bed and can also yield granular particles.

Other types of granulation techniques can be employed. Generally granulation is the action of obtaining solids in a granular form either by size enlargement or size reduction. In general size enlargement is employed. A good overview of the type of granulation processes available is described in W. Pietsch, "Size Enlargement by Agglomeration" (Wiley & Sons, 1991, page 385). Within this there are many different techniques available for granulation and this includes several agglomeration methods, which will be described. Here agglomeration results in small particles adhering to each other (agglomerating) to form larger particles (in this case the granular ones). Therefore, if a first technique results in the particles being too small an agglomerisation technique can then be employed to give bigger (granular) particles.

Tumble agglomeration is usually achieved using a tumbling, and/or rotating drum or cone drier with a powder having adhesive properties (so that the particles stick together). In some cases an extra added binder can be mixed. By this mechanism spherical particles can be formed.

Pressure agglomeration is usually characterised by high forces acting on a mass of a particulate matter. In general this process is performed with fine powders or with 'plastic' (non-elastic) materials. This process is normally used for powdered materials. (However it is also used in dried yeast production for doughs of a certain consistency). The shaped particles may be dried to suitable dry matter content for optimal storage. Pressure agglomeration can be accomplished by a piston, roller, isostatic and/or extruder presses. A good description of this type of equipment is given in the Pietsch book mentioned above.

Extrusion presses usually make use of wall friction, causing resistance to the flow of the plastic material through bores or open ended dies. Particularly in screw extruders extensive mixing takes place and high shear forces are applied.

In general materials with low melting or plastification temperatures can be directly agglomerated.

Other agglomeration techniques are possible. For example, spray drying in combination with a fluid bed agglomerator. Initially the biomass can be dried by atomization through a nozzle or using a rotary wheel in a spray dryer. Fine particles are recycled to the spraying section. The resulting sticky powder is further agglomerated in a fluid bed section. In some cases rewetting of the powder can improve the agglomeration process. This described technique is known as multi-stage drying.

To describe multi-stage drying in greater detail, the biomass is first spray dried. This can give a fine powder. The temperature of spray drying (air inlet temperature) is usually from 160 to 260°C and/or the air outlet temperature is from 75 to 90°C. Here the biomass is sprayed by a fast rotating disk or a nozzle which generates small particles. The particles can then fall, under gravity, towards the bottom of a spray drying tower. Here, a fluid bed may be provided, which can use hot air to effect drying (suitably at 90 to 95°C). Here, agglomeration can take place, and the particles can stick together. Following this, the agglomerated (granular) particles are subjected to drying, for example on a belt drying bed or on a sub-fluidised bed. At the start of the process, a biomass can have a dry matter content of below 30%. After spray drying, this can increase to from 75 to 90%, and after agglomerisation can be from 90 to 95 %. After drying, this can increase to at least 95%.

Another technique is to use a fluidised bed agglomerator. Here, powder can be fluidised in a gas flow. In the particle bed a fluid is sprayed with water that wets the powder and enhances the agglomeration.

In general the described agglomeration processes are for dry powders that can be plasticized. An exception is the drying on a multi-stage dryer. This combination of spray drying in combination with a fluid bed after dryer is suited for the agglomeration of many different types of biomass. However the process is not always suitable for thermo-labile products or products susceptible to oxidation by (hot) air. A good way of producing a granulated dry biomass is the extrusion of a mechanically dewatered filtercake followed by a suitable drying step like fluid bed or sub-fluidised bed drying.

Another way of agglomeration of (dried) biomass can be performed by the rewetting of (spray) dried product followed by an extrusion step and re-drying in e.g a fluid bed dryer. Powders, with a low melting point or a low plasticising temperature (or in case of certain dried biomasses with a high amount of intracellular oil, that partially melts due to the forces in the extruder) can be extruded. Suitable pellets form in the die plate.

As in (c) above, the (extruded or otherwise) granulated biomass can be dried, suitably under conditions that allow the particles to remain intact. The particle structure and size of the biomass after the granulation process is thought to enable the efficient drying of the biomass. The drying can be performed using various dryers, e.g. a belt dryer, a vacuum or a vacuum belt dryer, a fluidized or a subfluidized bed dryer. The skilled person can choose between a batch or a continuous process.

The use of a fluidized or subfluidized bed dryer is especially preferred in the process of the invention. Drying can occur in air or under nitrogen. With fluidized and subfluidized bed drying, the temperature in the bed can be adjusted to preset values. These values can range widely, for example from 35° to 120° C, such as 50 to 90°C, optionally from 60 to 80°C. If a labile compound needs to be isolated from the biomass, the temperature of the drying process can easily be adjusted to the lower ranges, to diminish the risk of oxidation or degradation.

Alternatively or in addition a vacuum drying process can be employed, e.g. at from 1 to 2 hours.

Several advantages may flow from the drying step. First, drying of the biomass particles (to form granules) can result in an intermediate material which may be stably stored for a prolonged time period. Here a (relatively) high dry matter content of the biomass may prevent degradation of the compound to be isolated from the biomass. In this way, the dried granules can be considered as a stable formulation of the compound present within or associated with the biomass.

For instance, the granules can function as a carrier for an enzyme, whereby the enzyme is immobilized within the granules by mixing an appropriate amount of a cross-linking agent, e.g. glutaraldehyde, into the biomass before extrusion.

In addition, the dried granules prepared according to the invention can be advantageously used as it is, for instance as a food or feed composition or additive.

The particles and/or granules (e.g. produced by extrusion) can have the following properties.

The granules can have the shape of chocolate confetti. The diameter of the (extruded) granules can vary from 0.1 to 12 mm, such as from 0.3 to 10 mm. More preferred is from 1.5 mm to 6 mm and optimally (for extraction when dried) the diameter is from 2 to 3 mm. The length of the granules can be about 2 to 5 or 6 times the diameter. They can then be easily handled in packing and used with commercially available extractors (to guarantee the permeability of the bed). Usually most, if not substantially all, the granules will have the same size, indeed, one can obtain highly uniform or homogeneous granules where at least 80%, such as at least 90%, of all the granules have a particular property within the range specified.

The granules are preferably free-flowing. They maybe roughly cylindrical in shape. This can be achieved by using extrusion. The particles can then be of a diameter that is approximately the same (although it may be slightly larger) than the holes of the die plate used for extrusion. During this process, particles may form automatically on exiting the die plate. In that event, the length of the particles will be variable. However, particle length can be influenced for example, if one uses a cutting means, for example a knife (e.g. one or more rotating blades adjacent to the die plate) when most (if not all) of the particles will have substantially the same length. Preferred lengths of such particles are at least 2 mm, such as at least 3 mm. Suitably the granules are of a size and water content that allows them to be "poured" which allows them to be stored and transported more easily. Although, generally speaking, most particles will be elongate in nature, some may be approximately spherical. The preferred lipid content of the granules is preferably from 30 to 50% by weight.

The bulk density of the granules will usually be from 400 to 1100 kg/m³.

As has been discussed, the granules are preferably porous, in order to allow access of the solvent to the compound to be extracted. Preferably, the granules have hollow channels, and these may extend towards, and into, the centre of the granules. The number of channels may be such that from 40 to 60% such as from 45 to 55%, optimally about 50%, by volume of the granule is hollow (air). As far as the channels are concerned, they may be in length 10 to 20 times that of their average diameter. The granules will, generally speaking, be homogeneous in their composition, in that the outside of the granule, will in essence, be the same material as that in the centre. This is in contrast to prior art yeast compositions which may have a relatively solid outside but yet relatively airy core.

The granules can be stably stored at a temperature optimal for the compound to be eventually extracted.

The preferred dry matter content of the dried granules is more than 80 %, more preferably at least 85 %, mostly preferably at least 90% and optimally in the range of from 93 to 97 %. If a water miscible solvent is to be used for extraction granules with lower dry matter contents can be used.

The (dried) granules are thus usually porous so solvents used in extraction can gain easy access to the (inside of) the granules. Thus, during extrusion and drying the amount of dust can be minimised (which increases yield) and can avoid an additional filtration of the (solvent) extract prior to evaporation of the extract.

The porosity of the granules is dependant on the (water or) dry matter content of granular particles. Often the water in the granular particles will be evaporated on drying to leave a (hollow) pore. The porosity of the dried granules is preferably from 15 to 50%, such as from 20 to 40%, optimally from 25 to 35%. Measurements of porosity are described later (Example 25).

Preferably, most (if not substantially all) of the cells in the granules are intact (that is to say not ruptured). The granules especially from a fungal biomass, can be wholly biomass particles which have a diameter from 0.3 to 10 mm, preferably a diameter of from 0.7 to 5 mm, optionally from 1 to 3 mm. Commonly, the particles will automatically form at the desired length. Otherwise, the particles may be cut to the desired length. If granulation was by extrusion, then the holes in the die plate of the extruder can generally correspond to the diameters of the granules.

Optionally, antioxidants may be added prior to or during the granulation process. These can include tocopherol and ascorbyl palmitate, e.g. present at up to 0.1% (by weight).

The invention may thus provide a biomass material with characteristics that may enable a cost-effective and efficient extraction of compounds. The compound(s) present can then be purified, isolated or (preferably) extracted. The process of the invention can enable the use of a percolation extraction process. The advantage allowed by this extraction process seem to be due to the structure and size as well as a high dry matter content. A dry extrudate requires a reduced amount of solvent for the extraction of the valuable compound therefrom. In addition, the process of desolventizing toasting, i.e. the release of used solvent from the biomass, can be performed better and more efficient with biomass in the form of an extrudate.

The extrudate residue obtained after the process of desolventizing toasting can advantageously be used as a feed component.

A dry matter content of the extrudate exceeding 90 to 95% may enable stable storage of the extrudate, whereas a dry matter content above 85% already can give a significant advantage in the subsequent extraction process.

Extraction is preferably conducted using a solvent. The solvent employed will depend upon the compound to be extracted, but in particular one can mention C₁₋₁₀ alkyl esters (e.g. ethyl or butyl acetate), toluene, C₁₋₆ alcohols (e.g. methanol, propanol) and C₃₋₈ alkanes (e.g. hexane) and/or a supercritical fluid (e.g. liquid CO₂ or supercritical propane). In prior art techniques, the solvent has been employed directly on the microorganism in the broth. However, by performing extraction on the granules, one can significantly reduce the amount of solvent required. In some of the applicant's experiments, 20 to 30 times less solvent was needed in order to perform the extraction. Not only does this result in a significant economic saving, because less solvent but is used, it also minimises emission problems. By using granules the surface area available to the solvent can be particularly high and therefore one can obtain good yields.

If the compound to be extracted is hydrophobic, then an apolar solvent is preferably used. For hydrophilic compounds, a polar solvent (such as a alcohol) is suitably employed.

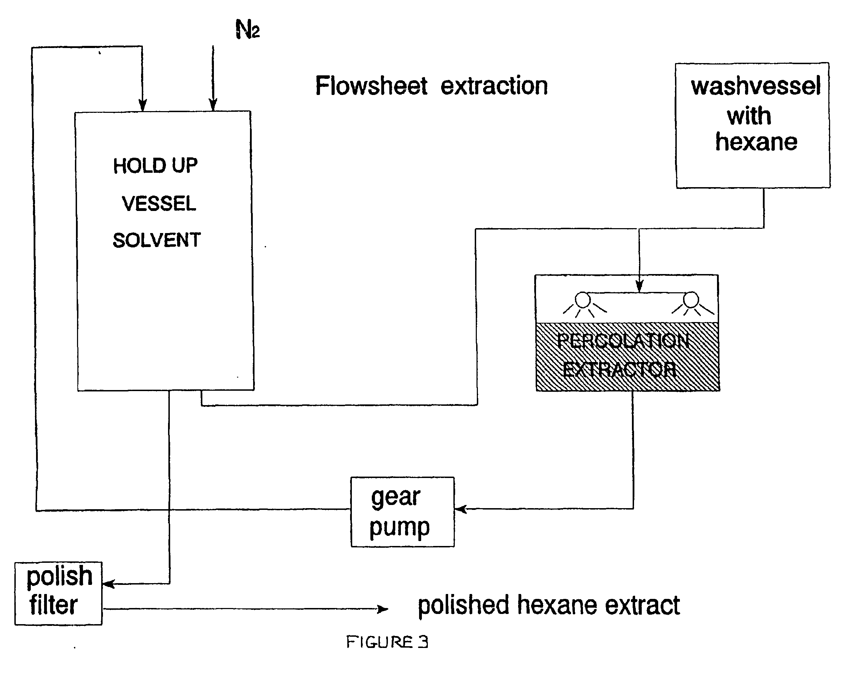

Extraction can be effected using a variety of techniques. The preferred method is percolation extraction, using a filter. Here, a column can be filled with the dried granules. The solvent (hexane) is then added to cover the granules. Although the solvent can be passed once through the column and over the dried granules, preferably it is recirculated (either as a closed or open system). Suitably the solvent is recirculated for three to seven times, such as about five times, suitably for a time period of from half an hour to one and a half hours such as about one hour. Figure 3 shows a suitable percolation extraction apparatus. The solvent is held in the vessel before addition to the percolation extractor containing the dried granules. The solvent is circulated by means of the pump. The polish filter is intended to remove fines.

Other percolation extractors can be employed. These may be of a counter current or cross-current design. In the former, the dried granules can be held in a rotating cylinder (such as a carousel) split into various sectors. The solvent is passed through the granules in one sector in one direction, and then passed through (preferably in the same direction) granules in another (such as a neighbouring) sector. These machines are often referred to as carousel extractors and are available from Krupp, Germany.

In another technique, the granules can be placed on, for example, a moving (e.g. porous) belt or conveyer which is moving in a substantially opposite direction to the solvent. This can mean that fresh granules are extracted with solvent that has already passed through other granules, and that fresh solvent is applied to granules that have previously been subjected to extraction with the solvent. This arrangement can maximise efficiency.

In a cross-current technique separate batches of the granules are subjected to extraction with portions of fresh solvent.

The process of the invention can also be used to obtain a mixture of two or more compounds from different microorganisms by preparing granular particles or granules from a mixture of two or more microorganisms. This mixture of microorganisms can be obtained by mixing the fermentation broths of two or more different microorganisms directly after has finished or by combining the biomass from two or more microorganisms immediately prior to the granulation (e.g. extrusion process). It is also possible to mix two or more different microbial extrudates prior to the extraction process.

A preferred process according to the present invention may thus be as follows:
a) fermenting one or more microorganisms in a suitable medium, under conditions that allow the microorganism to produce the desired compound, which can result in a broth (of the microorganisms in the surrounding medium) ;
b) if necessary, precipitating or solidifying the compound, such as by acidification;
c) separating the microorganisms from the medium in the broth, which may be achieved by solid/liquid separation, such as by filtration, in order to obtain a biomass;
d) pasteurisation, either of the broth resulting from (a) or of the biomass resulting from (c);
e) if necessary, increasing the dry matter content of the biomass, for example by adding dry matter or substances, or by decreasing the water content, for example by a dewatering or drying technique;
f) crumbling and/or kneading the resulting biomass (and, optionally, increasing the dry matter content by adding one or more dry substances);
g) granulating the biomass to give granular particles, such as by extrusion;
h) drying the granular particles to give dried granules; and
i) extracting one or more of the compounds, such as by using a suitable solvent.

The compounds isolated according to the invention can be of high quality and may be suitable for use in human or animal nutrition. Especially polyunsaturated fatty acid(PUFA)-containing lipids isolated according to the invention are suitable for nutritional purposes, in particular for the incorporation in infant formula.

The invention will now be described, by way of example, with regard to the following Examples which are provided by way of illustration. They are accompanied by the following drawings in which:
Figure 1 is a graph of temperature and dry matter (%) against time showing the drying behaviour of different amounts of extruded biomass at different temperatures;
Figure 2 is a graph of oil yield against temperature showing from extruded biomasses at different temperatures;
Figure 3 is a flow diagram of a (known) percolation extraction process;
Figure 4 is a graph of oil yield against time showing the relation between the amount of oil extracted and its time of extraction; and
Figures 5 and 6 are graphs of size distribution of dried granules according to the invention and prior art granules, respectively.

### EXAMPLES 1 TO 6

### Processing of Mortierella fermentation broth

160 1 of a fermentation broth of *Mortierella alpina,* previously pasteurised (68°C for 1 hour) (palletized growth) was filtered in a standard Dieffenbach plate and frame filter press (cloth type: nycot 2794). The broth was filtered with a maximum applied pressure of 1.0 bar. Within 20 minutes 160 1 broth was filtered over a total filter area of 4.35 m², which resulted in an average flow of about 110 l/m²h. The filter cake was washed with about 3 cake volumes (≈ 150 l) of process water.

About 30 kg of wet cake was recovered with a dry matter content of about 25 %. Three types of drying procedures were employed.

### Vacuum drying:

10 kg of filtercake was dried under vacuum at 35 °C in a vacuum (about 50 mbar) tray dryer (about 1 m² drying surface) during 24 hours resulting in about 2.5 kg of dried biomass with a dry matter content of about 94 %. The dried biomass consisted of crumbled biomass and some big lumps. Vacuum drying was time consuming probably due to the big lumps.

### Ventilation tray dryer:

10 kg of filtercake was dried under nitrogen during 24 hours at 35 °C in a ventilation tray dryer (about 1 m² drying surface). In total about 2.5 kg of dried biomass was recovered with a dry matter content of about 93 %. The dried biomass consist of crumbled biomass and some big lumps. Ventilation tray drying was time consuming probably due to the big lumps.

### Fluid bed dryer:

5 kg of filtercake was dried in a labscale fluid bed dryer of AEROMATIC (type MP-1) at an inlet air temperature of about 200 °C. The outlet temperature was about 40 °C. In about 45 minutes the wet biomass was dried resulting in about 1 kg of dried biomass with a dry matter content of about 81 %.

The dried material recovered by this last method was used for extraction of oil by means of hexane at six different temperatures (hence Examples 1 to 6). 150 g of the dried biomass was subjected to extraction with 1500 ml of hexane (heated to reflux) under nitrogen blanketing for 90 minutes. The cell mass was filtered off and the solvent in the resulting micella was evaporated in a rotavapor under vacuum. This resulted in a crude PUFA oil. The results are shown in Table 1. Extraction at room temperature gave lower yields; better yields were obtained at elevated temperatures.

**Table 1**

| Extraction of oil from biomass. | | | | |
|---|---|---|---|---|
| Experiment number | Biomass/hexane ratio | Temperature in °C | Extraction time in minutes | g oil per 100 g dried biomass |
| 1 | 300 | 80 | 30 | 19.2 |
| 2 | 100 | 23 | 30 | 16.4 |
| 3 | 150 | 45 | 60 | 22.6 |
| 4 | 200 | 23 | 120 | 17.1 |
| 5 | 200 | 23 | 30 | 11.8 |
| 6 | 100 | 23 | 120 | 13.5 |

The triglyceride rich oil was a light yellow oil, and contained some solid material.

### EXAMPLE 7 AND COMPARATIVE EXAMPLE 8

### Processing of Mortierella fermentation broth

500 1 of broth (previously pasteurised as described in the previous Example) was filtered in a membrane filter press (SCHULE) at a pressure difference of about 0.5 bar. The filtercake was washed with 10 cake volumes of process water and afterwards squeezed during 30 minutes at 5.5 bar. The resulting cake had a dry matter content of about 46 %. The cake recovered in this way was extruded in a pilot extruder (ODEKERKE, diameter barrel of 50 mm, barrel profiled). The die-plate had 10 holes with a diameter of 1.6 mm each. In total 19 kg of filtercake was extruded in about 45 minutes.

The extrudate recovered in this way was dried in pilot plant fluid bed dryer (T4 AEROMATIC 0.26 m² drying surface). Within about 45 minutes the extrudate was dried at 65 °C, resulting in a dry matter content of about 85 % (Example 7).

During the same experiment some filtercake was not extruded (Comparative Example 8) and dried in a vacuum tray dryer at 40 °C. The drying was very time consuming due to the big lumps.

Both materials were subjected to extraction using hexane. The following characteristics of the materials found:
- Dried extrudate: (Example 7): mainly pellets extraction process reasonably easy
- Vacuum dried biomass: (Comparative Example 8): pellets and lumps, much fines extraction process difficult; poor filtration properties

### EXAMPLES 9 and 10

Extrusion experiments using the same broth from Example 7 were performed using the following extruders:

### LALESSE (Arnhem, Netherlands):

In Example 9 a LALESSE single screw universal extruder was used. This type of extruder is normally used in the production of food snacks. Ground maize(dry matter content of about 95 %) was first fed as a test to the extruder and under pressure and heat the maize was extruded; once out of the die the extrudate expanded.

The barrel of this type of extruder was a profiled barrel in order to transport the maize processed. The type of screw used in extrusion is dependent upon the type of material processed. The screw was a universal transport screw or a compression screw with a diameter of 48 mm. The LALESSE machine is a 7.5 Kw pilot machine (drive on capacity). The total power requirement of the machine is 12.1 Kw. The barrel of the extruder could be heated or cooled. Dieplates with 1 up to 4 holes with diameters of 1.8, 2.0 and 2.2 mm used during extrusion of biomass.

The capacity for extending the *Mortierella* biomass (cooled barrel) was about 40 kg/h. In the extrusion the length/diameter (L/D) ratio of the hole in the die-plate was varied.

### ALMEX (Zutphen, Netherlands):

In Example 10, using the *Mortierella* biomass of Example 7, an expander extruder from the company ALMEX was used. This type of extruder is used in the production of pet-food. It had a smooth barrel with pins that enabled transport of the biomass. These pins have the same function as the profiles in the barrel of the LALESSE extruder. The screw of the expander extruder was a modular screw.
- Technical data:: ALMEX Contivar 150 L/D of 10 (ratio of the length of the screw and the diameter of the screw) Max. screw speed of 180 rpm 22 Kw (drive on capacity) Diameter screw of 150 mm Cooling with tapwater Die plates: 3 rings of holes with each hole a diameter of 1.8 mm

The biomass was raised to about 25 °C in temperature during processing. The capacity of the machine was about 250 kg of *Mortierella* extrudate per hour.

### COMPARATIVE EXAMPLE 11

### Comparison of solid/liquid separation performed with different methods

### Decanter:

350 1 of broth obtained from a fermentation of *Mortierella alpina* was decanted in the 'FLOTTWEG' decanter (type Z 23-3/441). The speed was set at about 4000 rpm. The differential speed range was varied during operation from 7.5 - 20 rpm.

The feed was set on 400 l/h. The biomass was not washed. In total 350 l broth was decanted. The temperature of the feed was 8 °C and of the supernatant 15 °C. The dry matter content of the recovered biomass was about 25 %.

### Decanter + vacuum drum filter:

20 kg of the biomass from the decanter experiment above with a dry matter content of 25 % was suspended in 500 l process water in which 10 kg NaCl was dissolved. The resulting slurry was filtered on a vacuum drum filter with belt discharge (PAXMAN, cloth type: 865.912 K/5 polyprop) without further washing. The speed of the drum was set on 1 rpm and the pressure difference on a maximum of 600 mbar. In total 400 1 was filtered within 15 minutes. The net filtering surface was about 0.3 m², which resulted in an average flow of 5000 l/m²h (filtering surface). The filtration rate was very well but the 'cake building' was rather bad. The dry matter content of the recovered filtered biomass was about 35 %.

### Plate and frame filter press:

500 l of broth was filtered in a plate and frame filter press (standard R&B, cloth type: nycot 2794). The broth was filtered with a pressure difference of 0.3 bar. Within 35 minutes 500 1 broth was filtered over a total filter area of 5 m², which resulted in an average flow of ± 175 l/m²h. The filter cake was washed in 30 minutes with about 2.5 cake volumes of process water which resulted in an average flow of 400 l/m²h.

The cake was blown dry by air for 30 minutes, which resulted in a dry matter content of the recovered biomass of about 25 %.

### Membrane filter press:

700 l of broth was filtered in a membrane filter press (SCHULE, cloth type: propex 46K2). The broth was filtered with a pressure difference of 0.3 bar. Within 30 minutes 700 1 broth was filtered over a total filter area of 6.8 m² which resulted in an average flow of about 205 l/m²h.

The filter cake was washed in 7 minutes with 3 cake volumes (≈ 300 l) of process water, which resulted in an average flow of 375 l/m²h.

The advantage of a membrane filter press over a plate and frame press is that the cake after filtration can be squeezed at high pressure, so the dry matter content of the cake will increase. The cake was squeezed at 5.5 bar during 30 minutes which resulted in a dry matter content of the recovered biomass of about 45 %.

In another experiment 1100 l of broth was filtered in a membrane filter press (SCHULE, cloth type: propex 46K2). The broth was filtered with a pressure difference of 0.3 bar. Within 45 minutes 1100 1 broth was filtered over a total filter area of 12.3 m² which resulted in an average flow of about 120 l/m²h. The filter cake was washed in 18 minutes with 3 cake volumes (≈ 600 1) of a 1 % NaCl solution, which resulted in an average flow of 162 l/m²h.

The cake was squeezed at 6 bar during 30 minutes, which resulted in a dry matter content of the recovered filtercake of about 55 %.

Both squeezing as well as washing of the cake with a 1 % salt solution had a significant effect on the dry matter content of the filtercake.

### EXAMPLE 12

### Extrusion of biomass with different dry matter contents

Extrusion was performed with biomass with different dry matter contents, which were obtained by the method presented in Example 7 (see Table 2). Extrusion was performed using a single screw extruder with a profiled barrel and a universal screw. The dieplates applied in extrusion had a different number of holes and the diameters of the holes were in the range of 2 mm.

The diameter of the particles obtained after extrusion was about 2 mm.

The performance and extrudate quality is depending on the percentage dry matter of the biomass used for extrusion. Although a 25% dry matter gave the poorest results, for other microorganisms such a low dry matter content can be acceptable.

**Table 2.**

| Results of extrusion experiments with biomass with different dry matter contents. | | |
|---|---|---|
| % Dry matter | performance of extrusion | Quality of extrudate |
| 25 | bad | very sticky material |
| 35 | good | sticky material |
| 45 | very good | non sticky extrudate |
| 55 | very good | loose extrudate |

### EXAMPLES 13 AND 14 AND COMPARATIVE EXAMPLE 15

### Drying of conventional and extruded biomass of Mortierella alpina

### Vacuum drying:

Conventionally recovered biomass (Comparative Example 15, not extruded) was dried in a vacuum tray dryer but took about 50 hours at 40 °C. The drying was very slow because of lumps. The dry matter content of in this way dried biomass was about 92.5 %.

For comparison about 20 g of extrudate (from Example 11, Ø_{particle} of 2 mm) with a dry matter content of 55 % was dried on labscale in a rotavapor. The temperature of the waterbath was 68 °C and the applied pressure 40 mbar. The performance of the drying was reasonable, except that the dried biomass stuck to the wall and sweated a little oil. The dry matter content after drying was 92.3 %.

### Fluidized bed drying:

In Example 13 drying was performed with biomass (Example 1) at different temperatures. Where no pretreatment of the biomass has occurred, big lumps of biomass did not become completely dry. In this case the dried biomass was very inhomogeneous considering the particle size.

If the biomass was pretreated before drying by means of extrusion, the performance of drying substantially improved. In this case the particle size of the dried biomass was more uniform.

The conclusion of these results is that fluidized bed drying can be performed with different forms of isolated biomass, but that drying will be improved using an extrudate.

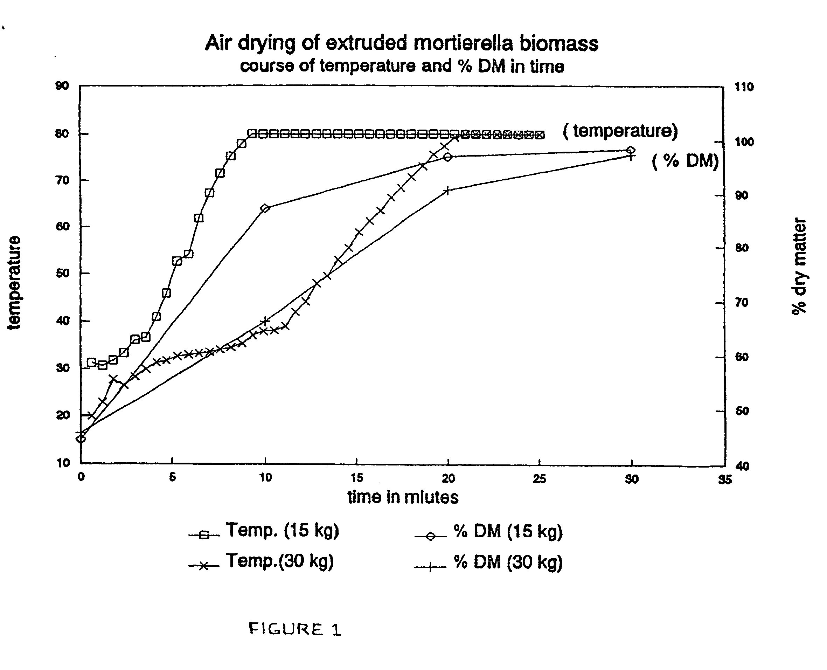

In another experiment (Example 14), drying of different quantities (15 and 30 kg) extrudate was performed in a fluidized bed dryer with air (8000 Nm³/m²h). During drying samples were taken and the dry matter content calculated. In Fig. 1 the relationship between temperature and dry matter content of the (two) different quantities is shown.

The bed temperature was set on 80 °C. The diameter of the extruded biomass was 1.3 mm. The dry matter content of the extruded biomass after drying was about 96%.

### EXAMPLE 16

### Extraction of lipid from dried extrudate of Mortierella alpina

### Stirred extraction of dried extrudate at different temperatures:

Samples of 100 g of dried extrudate with respectively 93.4 and 97.8 % dry matter were extracted during 3 hours with 500 ml hexane or 500 ml propanol-2, at temperatures of 20°, 35° and 50° C for hexane and 20°, 40° and 70° C for propanol-2. The slurry was stirred by means of a two blade stirrer in a 'four-necked' round bottom flask and heated by means of a heating mantle. Eventually evaporated hexane or propanol-2 was recycled by means of a reflux cooler.

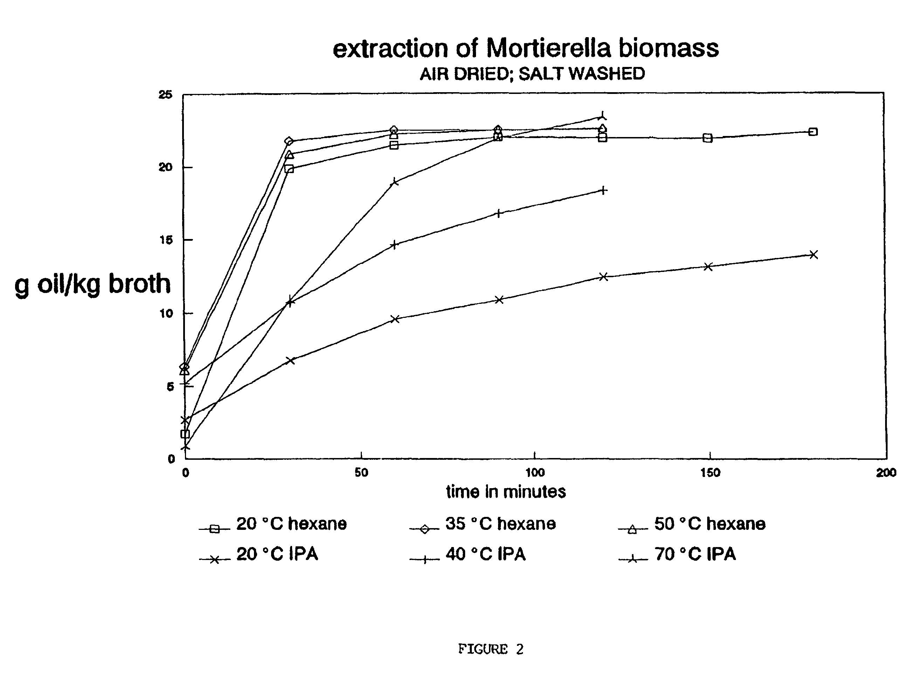

During the extraction, every 30 minutes a 15 ml sample of the supernatant was taken from the flask after the stirrer was stopped and the particles had settled. 1 ml of the samples was pipetted into preweighed 2 ml eppendorf tubes. After overnight drying under vacuum at 40 °C the eppendorf tubes were weighed and total oil was calculated. The results of the experiments are shown in Fig. 2.

### Conclusion for hexane extraction:

- the temperature had no effect on the total amount of lipid that can be extracted, i.e. a relatively low extraction temperature gives a good yield of lipid,
- the temperature had only a small effect on the time in which the total amount of lipid can be extracted,
- the total amount of lipid was extracted within 30 minutes from the biomass, with 5 volumes of hexane at a temperature above 20 °C.

### Conclusion for propanol-2 extraction:

- the temperature had a significant effect on total amount of lipid that can be extracted,
- the temperature had a significant effect on the time in which the total amount of lipid can be extracted,
- the total amount of lipid was extracted within 2 hours from the biomass with 5 volumes of propanol-2 at 73 °C.

The composition of the oil depended on the solvent used in extraction (see Table 3). The more polar the extraction solvent the more phospholipids were extracted. The polarity of the solvent can be chosen to optimise the composition of the oil.

**Table 3**

| Extraction of dried *Mortierella* biomass at room temperature using two different solvents. | | |
|---|---|---|
| Substance | hexane oil | propanol-2 oil |
| Tri-glycerides | 93 % | 85 % |
| di-glycerides | 2 % | 2 % |
| mono-glycerides | 2 % | 2 % |
| sterols | 3 % | 3 % |
| phospholipids | 2 % | 6.5 % |

On a larger scale problems were observed with the filtration of the micella, due to disintegration of the extrudate into small particles due to the high stirrer speed during the extraction process.

These problems were avoided using percolation extraction instead of stirred extraction.

### Percolation extraction of dried extrudate with hexane:

Several percolation extractions were performed on pilot scale (see Fig. 3 for a diagram of the process). About 40-45 kg of dried extruded biomass was extracted with hexane (initial hexane/biomass ratio of 4.4 l/kg) at 20 °C. The flow of the gear pump was set on 1.5 m³/h. There was a small nitrogen purge on holdup vessel of about 0.1 bar.

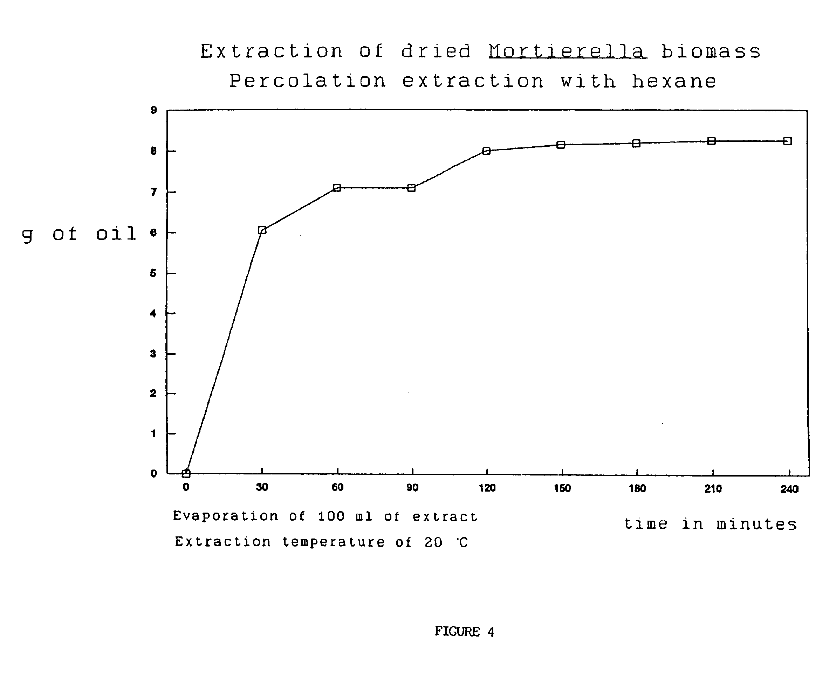

The extraction was performed during 4 hours (temperature increase during extraction from 18 to 25 °C). Each 30 minutes samples were taken from the micella. Of each sample, 100 ml was evaporated at labscale in a rotavapor (T_{waterbath} was 64 °C) during 20 minutes under vacuum (about 50 mbar). The amount of oil was estimated. The results are presented in Fig. 4. It can be noticed that after 2 hours an 'equilibrium' was reached. Afterwards, the extracted biomass was washed with about 0.6 bed volumes of hexane. During the extraction the bed height did not change.

The micella were polish filtered prior to evaporation. During the extraction we noticed that the micella became more and more clear, due to depth-filtration over the bed of particles.

### EXAMPLE 17 AND COMPARATIVE EXAMPLE 18

### Recovery of β-carotene oil from Blakeslea trispora

10 l of a fermentation broth of the fungus *Blakeslea trispora,* previously pasteurised (75°C for 15 minutes), was harvested using laboratory filtration equipment. To improve the filterability of the broth CaCl₂ was added (end concentration of 5 g/l). In this way recovered biomass was mechanically dewatered (squeezed) at labscale up to a 45% dry matter content using a typical fruit press (citrus press, HAFICO D.G.M)). The cake recovered in this way was extruded by means of a syringe of stainless steel equipped with a die-plate with 4 holes of 1.8 mm diameter each. The resulting extrudate was dried in a labscale fluid bed dryer (Tₐᵢᵣ = 40 °C, drying time of 90 minutes, airflow of 150 Nm³/h, AEROMATIC MP-1). The dry matter content of the biomass dried in this way was about 95 %.

A sample of about 50 g of dried extrudate was extracted using percolation extraction with ethyl acetate (initial volume/biomass ratio of 30 l/kg). After 2 hours of extraction at 50 °C the extract was harvested by means of vacuum filtration. The biomass was washed with 1 bed volume of ethyl acetate. The extract recovered in this way was washed twice with demineralised water (extract/water ratio of 5 v/v) prior evaporation. The ethyl acetate was evaporated at 50° C (T_{waterbath}) until a concentration of 8 g β-carotene/l was reached.

β-carotene crystals were recovered from the concentrate by means of controlled crystallisation and subsequent filtration.

The same experiment was performed with biomass that was blended and dried, and so not extruded (Example 18). The filterability after extraction of blended dried biomass was worse in comparison with dried extrudates.

### EXAMPLE 19 AND COMPARATIVE EXAMPLE 20

### Isolation of TAPS from dried extrudate from the yeast Pichia

Biomass from 2 l of a non-pasteurised fermentation broth of the yeast *Pichia ciferrii* was harvested by centrifugation at 5000 rpm during 10 minutes. The solid phase filtercake was washed with a salt solution containing 5 % NaCl. The biomass recovered in this way with 33 % dry matter was mechanically dewatered at labscale using a typical fruit press (HAFICO, D.G.M). During one minute the cake was dewatered at 200 kg/cm².

The dry matter content had increased to 47 %. The dewatered cake was extruded (Example 19) by means of a single screw laboratory extruder using a universal screw and a profiled barrel. The diameter of the hole in the dieplate was 2 mm.
The resulting extrudate was dried under vacuum during 40 hours at 40 °C, resulting in a dry matter content of about 92 %. 25 grams of the dried extrudate was extracted during 6.5 hours at 50 °C with ethyl acetate (ratio biomass/solvent of 1:5 (w/v)).

The extract recovered in this way was evaporated at 50 °C under vacuum (250 mbar), resulting in a TAPS oil (About 60 % Tetra Acetyl-Phyto Sphingosine/l oil).

Spray drying of TAPS-containing *Pichia ciferrii* (Comparative Example 20):

About 1 kg of broth of *Pichia ciferrii* was spray dried in a Büchi 190 laboratory spray dryer at an air temperature of 180 °C (inlet temperature) and 110 °C (outlet temperature). During the feed of the spray dryer the broth was stirred continuously. The broth was fed to the dryer at 1.2 l/h. The concentration of TAPS of the initial amount of TAPS was analysed in the spray dried product. In the spray dried product only 13 % of TAPS was recovered. Due to the high temperatures the TAPS was degraded. So this process is not an option for the isolation of TAPS due to the high process temperature.

### EXAMPLE 21

### Recovery of DHA oil from Crypthecodinium

Biomass from 7 l of a fermentation broth (previously pasteurised, 65°C for 1 hour) of the algae *Crypthecodinium cohnii* was harvested using a labscale centrifuge of the type BECKMAMN JM/6E. The broth was centrifuged in portions of 800 ml during 2 minutes at 5000 rpm resulting in a clear supernatant.

In total 224 g of biomass with a dry matter content of 13 % was recovered. This means a biomass concentration at harvest of the fermentation broth of about 4 g/kg. To this recovered biomass 300 g of starch (ROQUETTE, batch nr. 10EV0024)) was added to increase the dry matter content. The cake recovered in this way was extruded by means of a single screw lab extruder using a universal screw and a profiled barrel. The diameter of the hole in the dieplate was 2 mm and the thickness of the dieplate was 6 mm resulting in an L/D of the dieplate of 3. The resulting smooth extrudate was dried under vacuum overnight at 50 °C, resulting in a crackle dried extrudate. The dry matter content of the biomass dried in this way was about 94 %.

A sample of about 180 g of the dried extrudate was extracted with hexane (initial volume/biomass ratio of 5 l/kg). After 3 hours of extraction at 60 °C the micella was filtered over a Whatman filter. The resulting extracted biomass was washed once with 1000 ml of fresh hexane. The filtered micella recovered in this way was evaporated at 68°C (T_{waterbath}). In this way a crude DHA containing oil was recovered. The DHA concentration in the oil was 32.6 % analysed by means of GC. The in this way recovered oil contained about 67 % of tri-glycerides, 12 % di-glycerides, 3.7 % of sterols and about 0.2 % of antifoam (NMR). An other characteristic of the oil was the level of carotenoids (0.15 mg/ml of β-carotene and 5 mg/ml of γ-carotene).

### EXAMPLE 22

### Recovery of Vitamin B12 from Propionibacterium sp.

Broth from a large scale fermentation of a *Propionibacterium sp*. (28 tons) was harvested by means of a clarifier of the type BRPX-213-SGV(ALFA LAVAL, 3 - 7 tons /h) at a G-factor of about 5000. The broth clarified in this way was concentrated 2.5 times by means of ultra-filtration using a ABCOR KOCH module with about 150 m² spiral-wound poly ethylene sulphone membranes with a cut-off of 5 kD (type HFK 131-VSV). The resulting ultra filtrate was diafiltrated for 500 % according the concentrated volume with process water. The resulting diafiltrate was concentrated by a factor of 3 by means of vacuum evaporation, and the resulting concentrate heat shocked at 90°C for 2 minutes.

The resulting concentrate was granulated and dried in a NIRO 250 multi stage dryer (fluidised bed spray dryer/agglomerator). The inlet air temperature of the dryer had a temperature of about 250 °C and the outlet air temperature was about 70 °C. The air flow applied was about 3000 m³/h. This resulted in a product temperature of about 70 - 80 °C. The density of the concentrate fed to the dryer was about 1050 kg/m³.

A sample of about 2 g of dried granulate was used for extraction with 125 ml of about 75 % of ethanol (the water content gives an optimal extraction/technical performance) in a conical flask by means of stirring during 60 minutes at ambient temperature (clear extract). After extraction the extracted biomass was filtered using a Whatman paper filter (easy filtration). The clear pink filtrate recovered in this way was analysed for vitamin B12. The resulting biomass was washed with 25 ml of about 75 % ethanol. In this way about 90 % of the vitamin B12 was extracted from the granulated biomass (Table 4).

**Table 4**

| Data concerning extraction of vitamin B12 from Multi stage agglomerated *Propionic bacterium.* | | | | | | |
|---|---|---|---|---|---|---|
| sample number | | g | ml | density in kg/m³ | [vitamin B12] in mg/kg | total vitamin B12 in mg |
| VTB 9606^{E}/001 | input granulate | 2.01 | --- | --- | 842 | 1.69 |
| VTB 9606^{E}/002 | output extraction | 1.46 | --- | --- | 104.5 | 0.15 |
| VTB 9606^{E}/003 | extract | --- | 110 | 856 | 11.7 | 1.10 |
| VTB 9606^{E}/004 | wash | --- | 24 | 856 | 6.01 | 0.12 |

### EXAMPLE 23

### Co-extrusion C. cohnii and M. alpina

10 l of a fermentation broth of the fungus *Mortierella alpina* and 10 l of a fermentation broth of *Crypthecodinium cohnii* were mixed together. To improve the filterability of the mixed broth CaCl₂ was added (end concentration of 5 g/l). The mixed broth was filtered and the resulting cake was mechanically dewatered using a typical fruit press (citrus press, HAFICO).

The cake recovered in this way was extruded by means of a single screw lab extruder using a universal transport screw in a profiled barrel and a dieplate with one hole of 2 mm. The diameter of the extrudate was about 2 mm. The extrudate recovered in this way was dried in a labscale fluid bed dryer (Tₐᵢᵣ = 40 °C, drying time of about one hour , airflow of 150 Nm³/h, AEROMATIC MP-1). The dry matter content of the biomass dried in this way was about 92 %.

- A sample of about 100 g of dried extrudate was used for extraction with hexane (initial volume/biomass ratio of 4 l/kg). After 2 hours of extraction at ambient temperature the micella was recovered by means of vacuum filtration. The remaining extracted extrudate was washed with 4 volumes of fresh hexane (initial volume/biomass ratio of 4 l/kg). The washed hexane was mixed with the micella and the resulting micella was evaporated at 50 °C (T_{water bath}). In this way a crude PUFA oil was recovered containing ARA (C20:4 ω6) and DHA (C22:6 ω3).

The crude oil can be refined according methods usual for edible/vegetable oils.

### EXAMPLE 24

### Recovery of crude lovastatin from Aspergillus terreus

The pH of 7.5 l of a (non-pasteurised) fermentation broth of the fungus *Aspergillus terreus,* containing 2 g of lovastatin per kg broth, was adjusted to pH 2.1 with sulphuric acid to precipitate the lovastatin and stirred at this pH for 15 minutes. Subsequently the broth was filtrated, and the filtrate, containing only 3.5% of the total amount of lovastatin present in the broth, discarded.

The wet filter cake (230 gram, 18.5% dry matter) was mechanically dewatered to a dry matter content of 45% using a typical fruit press (citrus press, HAFICO), and subsequently extruded by means of a syringe of stainless steel equipped with a die-plate containing four holes of 1.8 mm each. The resulting extrudates were dried overnight at 40 °C in a vacuum oven to a dry matter content of about 95%. 10 g of the dried extrudates were extracted with 1 1 of a mixture of toluene (0.85 L) and acetone (0.15 L) during 15 minutes at ambient temperature. After separation of the extrudates by filtration, a lovastatin-containing extract was obtained with a lovastatin content of 1.5 g/l.

### EXAMPLE 25

### Recovery of ergosterol from commercial FERMIPAN

FERMIPAN is a commercially available dried yeast produced by *Gist brocades*. A FERMIPAN sample (batch nr. 9510702) of about 2 grams was extracted by means of 100 ml of 70 % ethanol during 30 minutes at ambient temperature. The resulting clear extract was separated from the extracted FERMIPAN by means of filtration. The clear filtrate recovered in this way was analysed by means of NMR. In about 22 g of the extract 0.09 mg of ergosterol was found. Important in the extraction of ergosterol is the water content in the ethanol. This concentration was not optimised. The experiment was an indicative experiment to show extraction of the valuable compound ergosterol from granulated (extruded dried yeast) yeast.

### EXAMPLE 25

### Advantages of granulated/extruded biomass

PUFA biomass was processed according the route described in Example 1 (filtration, extrusion and drying).

The porosity of the dried extruded PUFA biomass was measured on a porosimeter (Pharmitalia Carlo Erba, Italy) 2000 and a (Carlo Erba) macroporse unit 120. The results are presented in Table 5.

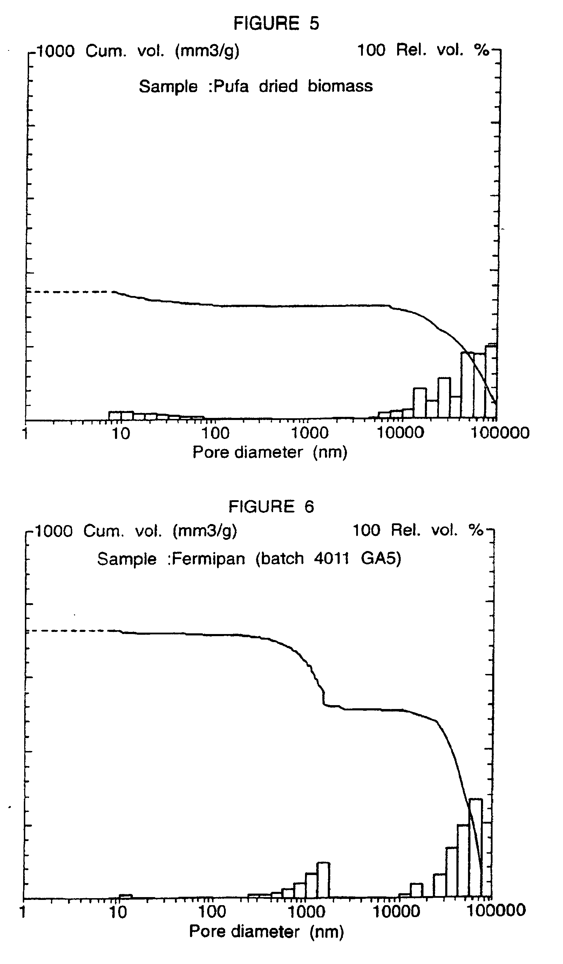

The pore-volume distribution is presented in Figure 5. Figure 6, for comparison, gives the pore-volume distribution of dried yeast (FERMIPAN, Gist-brocades).

The line (with the Y-axis left) displays the cumulative pore volume and the histogram (with the Y- axis right) is a relative differential pore volume distribution. It is shown that the majority of pores are found in the 10,000 - 100,000 nm pore-diameter region.

**Table 5 :**

| Results of porosity measurement (twice) of same PUFA extruded dried biomass (mercury porosity method). | | | | | |
|---|---|---|---|---|---|
| Sample | sample weight in g | sample volume in cm³ | sample density in g/cm³ | total pore volume in cm³/g | porosity in % |
| PUFA dried biomass I | 0.5008 | 0.555 | 0.90 | 0.304 | 27 |
| PUFA dried biomass II | 0.8684 | 1.011 | 0.86 | 0.348 | 30 |

81 g dried extruded PUFA biomass was subjected to extraction in a column by means of hexane at ambient temperature (20 °C) in a cross-current percolation extraction to obtain the PUFA oil. Thirteen bed volumes of fresh hexane (one bed volume is the quantity of hexane needed to drown the whole bed) were drained through the bed of biomass. The amount of crude oil in each bed volume drained hexane was determined by weight after evaporation of hexane.

### Conditions of extraction:

- the intra particle volume of the biomass bed was 80 ml (= 1 bed-volume)
- flow rate of 1 bed volume per 300 seconds.
- 50 % of the total amount of oil was extracted in 300 seconds in one bed volume
- the bulk-density of the dried biomass was 570 kg / m³.
- the particle diameter was 0.0025 m
- extraction temperature of 20 °C
- solubility of oil in hexane is infinite.
- oil content of biomass 35.5 w/w (%).

The hexane was evaporated at labscale in a rotavapor at 60 °C (T_{waterbath}) under vacuum to yield the PUFA. The PUFA particles (dried extrudates) as described were porous uniform particles, which were ideal for extraction. There was a large surface area available for exchange of oil. The diffusion limitation for extraction therefore is low.

### COMPARATIVE EXAMPLE 26

The various culturing conditions that were used to obtain the biomass and broths described in the previous examples will now be given in the following table.

| Micro organism | Product | Process type | Nutrients (g/l) | | Temperature (°C) | pH | Time (hrs) |
|---|---|---|---|---|---|---|---|
| *Pichia ciferrii* (*=Hansenula ciferrii)* | Tetra-Acetyl-Phyto-Sphingosine (=TAPS) (extracellular) | fed batch (glucose feed) | glucose: | 30 | 25 | 6.5 - 6.8 | 96 |
| | | | yeast extract: | 3 | | | |
| | | | malt extract: | 3 | | | |
| | | | peptone: | 5 | | | |
| | | | | | | | |
| *Mortierella alpina* | arachidonic acid (intracellular) | batch | glucose: | 50 | 25 | 5.5-7 | 120-168 |
| | | | yeast extract: | 5 | | | |
| | | | NaNO₃: | 5 | | | |
| | | | K₂HPO₄: | 3 | | | |
| | | | MgSO₄.7H₂O: | 0.5 | | | |
| | | | ammonium salt | | | | |
| | | | minerals | | | | |
| | | | | | | | |
| *Blakeslea trispora* | β-carotene (intracellular) | batch | Pharmamedia: | 75 | 26-28 | 6.5 | 7 days |
| | | | glucose: | 10 | | | |
| | | | KH₂PO₄: | 0.5 | | | |
| | | | MnSO₄.H₂O: | 0.1 | | | |
| | | | soybean oil: | 30 | | | |
| | | | cotton seed oil: | 30 | | | |
| | | | dextrins: | 60 | | | |
| | | | Triton X-100: | 1.2 | | | |
| | | | ascorbic acid: | 6 | | | |
| | | | lactic acid: | 2 | | | |
| | | | thiamine-Hcl: | 2mg | | | |
| | | | isoniazid: | 0.075% | | | |
| *Aspergillus terreus* | Lovastatin | fed batch (glucose + ammonia) | glucose: | 20 | 28 | 6.5 | 8 days |
| | | | yeast extract: | 11.3 | | | |
| | | | KH₂PO₄: | 3.5 | | | |
| | | | Na₂SO₄ : | 1.0 | | | |
| | | | MgSO₄. 7H₂O: | 1.4 | | | |
| | | | CaCl₂. 2H₂O: | 0.1 | | | |
| | | | PPG (2000) | | | | |
| | | | (antifoam): | 0.1 | | | |
| | | | trace elements | | | | |
| *Propionibacterium sp.* | Vitamin B₁₂ (intracellular) | fed batch | glucose: | 10 | 30 | 5-7.5 | 168 |
| | | (anaërobe, | corn steep liq.: | 80 | | | |
| | | (glucose | (NH₄)₂SO₄: | 16 | | | |
| | | feed + | KH₂PO₂: | 0.4 | | | |
| | | after 4 | Na₂HPO₄.12H₂O: | 1.5 | | | |
| | | days: | MgSO₄.7H₂O: | 0.5 | | | |
| | | 10mg/l | minerals | | | | |
| | | 5,6 - dimethylbenzimidazole) | Ca | | | | |
| | | | pantothenate: | 10mg | | | |
| *Crypthecodinium cohnii* | Docosahexaenoic acid (=DHA) (intracellular) | fed batch | Ocean^{®} | | 20-28 | 7-7.8 | 70-80 |
| | | (68g/l | artificial | | | | |
| | | glucose | seawater: | 250 | | | |
| | | + 24g/l | (instant) | | | | |
| | | yeast | yeast extract: | 6 | | | |
| | | extract) | glucose: | 12 | | | |

### REFERENCES CONCERNING FERMENTATION TECHNIQUES

Maister H.G., Rogovin S.P., Stodola F.H., Wickerham L.J., "Formation of Extracellular Sphingolipids by Microorganisms. IV. Pilot-Plant Production of Tetraacetylphytosphingosine by *Hansenula ciferrii*".

Appl. Microbiol., 10, 401-406. (1962) Zu-Yi Li, Yingyin Lu,Yadwad V.B.,Ward O.P.,"Proces for Production of Arachidonic Acid Concentrate by Strain of *Mortierella alpina*"

Can. J. Biochem. Eng. 73, 135-139 (1995)
Finkelstein M.,Huang C-C., Byng G.S.,Tsau B-R., Leach J., "*Blakeslea trispora* mated culture capable of increased beta-carotene production" US patent 5,422,247 (1995)

Kojima I., Kouji K., Sato H., Oguchi Y., "Process for the producing Vitamin B₁₂ by the fermentation technique, and Vitamin B₁₂-producing microorganism". US patent 4,544,633 (1985)

Kyle D.J., Reeb S.E., Sicotte V.J., "Production of decosahexaenoic acid by dinoflagellates". US patent 5,407,957 (1995)

## Claims

1. A process for the isolation of one or more compound(s) from a microbial biomass which comprises a microorganism that has produced such a compound, the process comprising:
a) providing, or obtaining a biomass with a dry matter content of from 25 to 80%;
b) extruding the biomass into granular particles having an average dry matter content of from 25 to 80%;
c) drying the granular particles to give porous dried granules having an average dry matter content of at least 80%; and
d) purifying, extracting or isolating the or each compound from the dried granules resulting from (c),
wherein
A) the biomass comprises, or originates from, a filamentous fungus or an algae or the microorganism is a bacteria; or
B) in d), the or each compound is extracted from the dried granules resulting from (c) using a solvent which is a C₁₋₁₀ alkyl ester, toluene, a Alcohol or a C₃₋₈ alkane.

2. A process according to claim 1 wherein the biomass is subjected to crumbling or kneading before granulation.

3. A process according to claim 1 or 2 wherein the biomass in (a) is obtained by solid/liquid separation performed on a fermentation broth and optionally the solid/liquid separation is combined with mechanical dewatering.

4. A process according to any preceding claim wherein in (a) the biomass with a dry matter content of 25 to 80% is obtained by:
i) the addition of a solid material to the biomass; or
ii) dewatering of the biomass.

5. A process according to any preceding claim wherein the drying of the granulated biomass in (c) to a dry matter content of at least 80% is performed by fluidized bed or subfluidized bed drying or vacuum drying.

6. A process according to any preceding claim wherein the biomass comprises, or originates from, a fungus or an algae.

7. A process according to claim 6 wherein the fungus belongs to the order *Mucorales*, and/or the algae is a dinoflagellate and/or belongs to the genus *Crypthecodinium.*

8. A process according to claim 6 wherein the fungus belongs to the genus *Mortierella*, *Phycomyces*, *Blakeslea or Aspergillus.*

9. A process according to any one of claims 6 to 7 wherein the fungus is *Mortierella alpine* and/or the algae is *Crypthecodinium cohnii*.

10. A process according to preceding claim wherein the compound is a polyunsaturated fatty acid (PUFA), optionally contained in a lipid.

11. A process according to claim 10 wherein the polyunsaturated fatty acid is a C18, C20 or C22 ω-3 or a C18, C20 or C22 ω-6 polyunsaturated fatty acid.

12. A process according to claim 11 wherein the compound is a C20 or C22 ω-3 or C20 or C22 ω-6 polyunsaturated fatty acid.

13. A process according to any preceding claim wherein the compound is arachidonic acid (ARA), eicosapentaenoic acid (EPA) and/or docosahexaenoic acid (DHA).

14. A process according to any one of claims 1 to 9 wherein the compound is a carotenoid or a HMG-CoA reductase inhibitor (e.g. lovastatin, pravastatin or compactin).

15. A process according to any preceding claim wherein the microorganism is a yeast and/or the compound is tetra-acetyl-phyto-sphingosine (TAPS).

16. A process according to any preceding claim wherein the microorganism is a bacteria and/or the compound is a vitamin.

17. A process according to the preceding claim wherein the biomass is obtained from a fermentation broth after acidification (such as to a pH <5).

18. A process according to claim 17 wherein acidification (e.g. to a pH of about 2) results in precipitation of the compound.

19. A porous microbial extrudate which comprises, or is obtained from, a fungus of the genus *Mortierella*, wherein the dry matter content of the extrudate is above 85%.

20. A process for the isolation of one or more compound(s) from granules of biomass, the process comprising
a) providing porous dried granules having a dry matter content of at least 80%, the granules having been derived from an extruded microbial biomass comprising microorganisms that have produced such a compound; and
b) extracting or isolating the or each compound from the dried granules by solvent extraction,
wherein
A) the biomass comprises, or originates from, a filamentous fungus or an algae or the microorganisms are bacteria; or
B) the extraction in b) is conducted using a solvent which is a C₁₋₁₀ alkyl ester, toluene, a C₁₋₆ alcohol or a C₃₋₈ alkane.

## Patentansprüche

1. Verfahren zur Isolierung von einer oder mehreren Verbindungen aus einer mikrobiellen Biomasse, die einen Mikroorganismus umfasst, der eine solche Verbindung produziert hat, das Verfahren umfassend:
a) Bereitstellen oder Erhalten einer Biomasse mit einem Trockensubstanzgehalt von 25 bis 80%;
b) Extrudieren der Biomasse in körnigen Partikel mit einem durchschnittlichen Trockensubstanzgehalt von 25 bis 80%;
c) Trocknen der körnigen Partikel zu porösen getrockneten Körnchen mit einem durchschnittlichen Trockensubstanzgehalt von mindestens 80%; und
d) Aufreinigen, Extrahieren oder Isolieren der oder jeder Verbindung aus den getrockneten Körnchen, die aus (c) resultieren;
wobei
A) die Biomasse einen Fadenpilz oder eine Alge umfasst oder davon stammt oder es sich bei dem Mikroorganismus um ein Bakterium handelt; oder
B) die oder jede Verbindung in d) unter Verwendung eines Lösungsmittels, bei dem es sich um einen C₁₋₁₀-Alkyloster, Toluol, einen C₁₋₆-Alkohol oder ein C₃₋₈-Alkan handelt, aus den getrockneten Körnchen, die aus (c) resultieren, extrahiert wird.

2. Verfahren nach Anspruch 1, wobei die Biomasse vor der Körnung einem Zerkrümeln oder Kneten unterworfen wird.

3. Verfahren nach Anspruch 1 oder 2, wobei die Biomasse in (a) erhalten wird durch Fest/Flüssig-Auftrennung, die in einer Fermentationsbrühe durchgeführt wird, und die Fest/Flüssig-Auftrennung wahlweise mit mechanischem Entwässern kombiniert wird.

4. Verfahren nach einem der vorherigen Ansprüche, wobei in (a) die Biomasse mit einem Trockensubstanzgehalt von 25 bis 80% erhalten wird durch:
(i) die Zugabe eines Feststoffes zu der Biomasse; oder
(ii) Entwässern der Biomasse.

5. Verfahren nach einem der vorherigen Ansprüche, wobei das Trocknen der gekörnten Biomasse in (c) zu einem Trockensubstanzgehalt von mindestens 80% durch Wirbelschicht ("fluidized bed" oder "subfluidized bed")-Trocknen oder Vakuumtrocknen durchgeführt wird.

6. Verfahren nach einem der vorherigen Ansprüche, wobei die Biomasse einen Pilz oder eine Alge umfasst oder davon abstammt.

7. Verfahren nach Anspruch 6, wobei der Pilz zur Ordnung Mucorales gehört und/oder die Alge eine Dinoflagellate ist und/oder zur Gattung *Crypthecodinium* gehört.

8. Verfahren nach Anspruch 6, wobei der Pilz zur Gattung *Mortierella*, *Phycomyces*, *Blakeslea* oder *Aspergillus* gehört.

9. Verfahren nach einem der Ansprüche 6 bis 7, wobei der Pilz *Mortierella alpina* ist und/oder die Alge *Crypthecodinium cohnii* ist.

10. Verfahren nach einem der vorherigen Ansprüche, wobei die Verbindung eine mehrfach ungesättigte Fettsaure (PUFA) ist, gegebenenfalls in einem Lipid enthalten.

11. Verfahren nach Anspruch 10, wobei die mehrfach ungesättigte Fettsäure eine C18-, C20- oder C22-ω-3- oder eine C18-, C20- oder C22-ω-6-mehrfach ungesättigte Fettsäure ist.

12. Verfahren nach Anspruch 11, wobei die Verbindung eine C20- oder C22-ω-3-oder C20- oder C22-ω-6-mehrfach ungesättigte Fettsäure ist.

13. Verfahren nach einem der vorherigen Ansprüche, wobei die Verbindung Arachidonsäure (ARA), Eicosapentaensäure (EPA) und/oder Docosahexaensäure (DHA) ist.

14. Verfahren nach einem der Ansprüche 1 bis 9, wobei die Verbindung ein Carotinoid oder ein HMG-CoA-Reduktase-Inhibitor (z.B. Lovastatin, Pravastatin oder Compactin) ist.

15. Verfahren nach einem der vorherigen Ansprüche, wobei der Mikroorganismus eine Hefe und/oder die Verbindung Tetraacetylphytosphingosin (TAPS) ist.

16. Verfahren nach einem der vorherigen Ansprüche, wobei der Mikroorganismus ein Bakterium ist und/oder die Verbindung ein Vitamin ist.

17. Verfahren nach einem der vorherigen Ansprüche, wobei die Biomasse aus einer Fermentationsbrühe nach Ansäuern (z.B. zu einem pH < 5) erhalten wird.

18. Verfahren nach Anspruch 17, wobei das Ansäuern (z.B. zu einem pH-Wert von ungefähr 2) zu einer Präzipitation der Verbindung führt.

19. Poröses mikrobielles Extrudat, welches einen Pilz der Gattung *Mortierella* umfasst oder daraus erhalten wird, wobei der Trockensubstanzgehalt des Extrudats über 85% liegt.

20. Verfahren zur Isolierung von einer oder mehreren Verbindungen aus Biomassekörnchen, das Verfahren umfassend:
a) Bereitstellen von porösen getrockneten Körnchen mit einem Trockensubstanzgehalt von mindestens 80%, wobei die Körnchen von einer extrudierten mikrobiellen Biomasse stammen, umfassend Mikroorganismen umfasst, die eine solche Verbindung hergestellt haben; und
b) Extrahieren oder Isolieren der oder jeder Verbindung aus den getrockneten Körnchen durch Lösungsmittelextraktion;
wobei
A) die Biomasse einen Fadenpilz oder eine Alge umfasst oder davon stammt oder es sich bei dem Mikroorganismus um ein Bakterium handelt; oder
B) die Extraktion in b) unter Verwendung eines Lösungsmittels, bei dem es sich um einen C₁₋₁₀-Alkyloster, Toluol, einen C₁₋₆-Alkohol oder ein C₃₋₈-Alkan handelt, durchgeführt wird.

## Revendications

1. Procédé permettant d'isoler un ou plusieurs composés à partir d'une biomasse microbienne contenant un microorganisme qui a produit un tel composé, lequel procédé comporte :
a) le fait de prendre ou d'obtenir une biomasse dont la teneur en matière sèche vaut de 25 à 80 % ;
b) le fait d'extruder cette biomasse pour en faire des particules granulaires contenant en moyenne de 25 à 80 % de matière sèche ;
c) le fait de faire sécher ces particules granulaires pour obtenir des granulés séchés poreux contenant en moyenne au moins 80 % de matière sèche; et
d) le fait de purifier, d'extraire ou d'isoler le composé voulu ou chacun des composés voulus, à partir des granulés séchés obtenus lors de l'étape (c),
dans lequel
A) la biomasse comprend, ou provient de, un champignon filamenteux ou une algue ou le micro-organisme est une bactérie ; ou
B) en d), le composé ou chaque composé est extrait des granulés séchés résultant de (c) en utilisant un solvant qui est un ester d'alkyle en C₁ à C₁₀, du toluène, un alcool en C₁ à C₆ ou un alcane en C₃ à C₈.

2. Procédé conforme à la revendication 1, dans lequel on fait subir à la biomasse, avant sa granulation, une fragmentation ou un malaxage.

3. Procédé conforme à la revendication 1 ou 2, dans lequel, dans l'étape (a), on obtient la biomasse en soumettant un bouillon de fermentation à une opération de séparation solide/liquide, séparation qui est éventuellement combinée avec une opération mécanique de déshydratation.

4. Procédé conforme à l'une des revendications précédentes, dans lequel, dans l'étape (a), on obtient la biomasse dont la teneur en matière sèche vaut de 25 à 80 % :
i) par addition d'une matière solide à la biomasse ;
ii) ou par déshydratation de la biomasse.

5. Procédé conforme à l'une des revendications précédentes, dans lequel, dans l'étape (c), le séchage des particules granulaires de biomasse jusqu'à une teneur en matière sèche d'au moins 80 % est effectué par séchage en lit fluidisé ou sous-fluidisé ou par séchage sous vide.

6. Procédé conforme à l'une des revendications précédentes, dans lequel la biomasse contient un champignon ou une algue ou en dérive.

7. Procédé conforme à la revendication 6, dans lequel le champignon appartient à l'ordre des *Mucorales*, et/ou l'algue est une dinoflagellée et/ou appartient au genre *Crypthecodinium.*

8. Procédé conforme à la revendication 6, dans lequel le champignon appartient au genre *Mortierella, Phycomyces*, *Blakeslea* ou *Aspergillus.*

9. Procédé conforme à l'une des revendications 6 et 7, dans lequel le champignon est *Mortierella alpina* et/ou l'algue est *Crypthecodinium cohnii*.

10. Procédé conforme à la revendication précédente, dans lequel le composé est un acide gras polyinsaturé (AGPI), éventuellement contenu dans un lipide.

11. Procédé conforme à la revendication 10, dans lequel l'acide gras polyinsaturé est un acide gras polyinsaturé ω-3 en C18, C20 ou C22 ou un acide gras polyinsaturé ω-6 en C18, C20 ou C22.

12. Procédé conforme à la revendication 11, dans lequel le composé est un acide gras polyinsaturé ω-3 en C20 ou C22 ou un acide gras polyinsaturé ω-6 en C20 ou C22.

13. Procédé conforme à l'une des revendications précédentes, dans lequel le composé est l'acide arachidonique (ARA), l'acide eicosapentaénoïque et/ou l'acide docosahexaénoïque.

14. Procédé conforme à l'une des revendications 1 à 9, dans lequel le composé est un caroténoïde ou un inhibiteur de la HMG-CoA réductase (par exemple, lovastatine, pravastatine ou compactine).

15. Procédé conforme à l'une des revendications précédentes, dans lequel le microorganisme est une levure et/ou le composé est une phytosphingosine tétra-acétylée (TAPS).

16. Procédé conforme à l'une des revendications précédentes, dans lequel le microorganisme est une bactérie et/ou le composé est une vitamine.

17. Procédé conforme à la revendication précédente, dans lequel la biomasse est obtenue à partir d'un bouillon de fermentation, après acidification, par exemple à pH inférieur à 5.

18. Procédé conforme à la revendication 17, dans lequel l'acidification, par exemple à pH d'environ 2, a pour résultat de faire précipiter le composé.

19. Extrudat microbien poreux, comprenant un champignon ou obtenu à partir d'un champignon du genre *Mortierella*, où la teneur en matière sèche de l'extrudat est supérieure à 85%.

20. Procédé permettant d'isoler un ou plusieurs composés à partir de granulés de biomasse, lequel procédé comporte :
a) le fait de prendre des granulés séchés poreux contenant au moins 80 % de matière sèche et provenant d'une biomasse microbienne extrudée comprenant des microorganismes qui ont produit un tel composé ;
b) et le fait d'extraire ou d'isoler le composé voulu ou chacun des composés voulus à partir des granulés séchés, par extraction par solvant,
dans lequel
A) la biomasse comprend, ou provient de, un champignon filamenteux ou une algue ou les micro-organismes sont des bactéries ; ou
B) l'extraction en b) est conduite en utilisant un solvant qui est un ester d'alkyle en C₁ à C₁₀, du toluène, un alcool en C₁ à C₆ ou un alcane en C₃ à C₈.
